# EUROPEAN PATENT SPECIFICATION

(11) **EP 3 921 428 B1**
(45) Date of publication and mention of the grant of the patent: **03.09.2025**
(21) Application number: 20705120.2
(22) Date of filing: 07.02.2020
(51) Int. Cl.: C12N 15/86, C12N 7/00, A61K 39/12

(54) **METHODS FOR PROVIDING PURIFIED VIRAL PARTICLES OF SEMLIKI FOREST VIRUS (SFV), PREPARATIONS OBTAINABLE THEREBY, AND USES THEREOF**
VERFAHREN ZUR BEREITSTELLUNG VON GEREINIGTEN VIRALEN PARTIKELN DES SEMLIKI-FOREST-VIRUS (SFV), DAMIT GEWONNENE PRÄPARATE UND DEREN VERWENDUNGEN
PROCÉDÉS D'OBTENTION DE PARTICULES VIRALES PURIFIÉES DE VIRUS DE LA FÔRET DE SEMLIKI (SFV), PRÉPARATIONS POUVANT ÊTRE OBTENUES PAR CE PROCÉDÉ, ET LEURS UTILISATIONS

(30) Priority: 08.02.2019 NL 2022538
(43) Date of publication of application: 15.12.2021
(73) Proprietor: Vicinivax Holding B.V., 9713 GX Groningen (NL); RIJKSUNIVERSITEIT GRONINGEN, 9712 CP Groningen (NL); Academisch Ziekenhuis Groningen, 9713 GZ Groningen (NL)
(72) Inventor: DAEMEN, Catharina Arnoldine Hubertina Henrica, 9713 AV Groningen (NL); SCHOEMAKER, Trieneke Jolanda, 9713 GX Groningen (NL); ZORZ, Mirjan, 1290 Grosuplje (SI); GASPERSIC, Jernej, 6250 Ilirska Bistrica (SI); JARC, Marko, 5220 Tolmin (SI); BANJAC, Marko, 5270 Ajdovscina (SI)
(74) Representative: V.O.
(86) International application number: PCT/NL2020/050067
(87) International publication number: WO 2020/162752

(56) References cited:
- EP-A1- 2 848 692
- WO-A1-2012/169970
- WO-A1-2014/151855
- WO-A2-01/92552
- WO-A2-2004/055167
- WO-A2-2006/039253
- JP-A- 2007 117 003
- DAEMEN T ET AL: "Immunization strategy against cervical cancer involving an alphavirus vector expressing high levels of a stable fusion protein of human papillomavirus 16 E6 and E7", GENE THERAPY, NATURE PUBLISHING GROUP, LONDON, GB, vol. 9, no. 2, 1 January 2002 (2002-01-01), pages 85 - 94, XP002590699, ISSN: 0969-7128, DOI: 10.1038/SJ/GT/3301627
- OMAR A ET AL: "One-step separation of the components of semliki forest virus by cation exchange chromatography", JOURNAL OF VIROLOGICAL METHODS, ELSEVIER BV, NL, vol. 12, no. 1-2, 1 October 1985 (1985-10-01), pages 71 - 83, XP023695896, ISSN: 0166-0934, [retrieved on 19851001], DOI: 10.1016/0166-0934(85)90009-6
- EDUARDO ANSORENA ET AL: "A simple and efficient method for the production of human glycosylated glial cell line-derived neurotrophic factor using a Semliki Forest virus expression system", INTERNATIONAL JOURNAL OF PHARMACEUTICS, vol. 440, no. 1, 1 May 2012 (2012-05-01), pages 19 - 26, XP055118644, ISSN: 0378-5173, DOI: 10.1016/j.ijpharm.2012.04.071

## Description

The invention relates to purified vaccine preparations and methods for providing them. In particular, it relates to methods for purifying Semliki Forest Virus (SFV) replicon particles expressing one or more antigens derived from a pathogen or tumor, such as a viral antigen and tumor antigens. Provided are methods for producing infectious virus particles or viral replicon particles in high yields, especially particles useful in immunotherapies, cancer vaccines, vaccines for infectious diseases and/or gene therapy applications. In particular, the present invention discloses a high-yielding, GMP-compatible, commercially feasible process for producing highly purified SFV replicon particle preparations suitable for use in human and veterinary medicine.

SFV belongs to the genus Alphavirus of the family of the *Togaviridae.* Alphaviruses include a nucleocapsid with one copy of a single-stranded RNA molecule surrounded by an envelope containing spike proteins. Alphavirus RNA has a positive polarity that enables the genomic RNA to initiate an infection when introduced into the cytoplasm of a cell. In addition, the RNA is self-replicating since it encodes its own replicase, and replication results in high-level expression of the viral proteins in host cells.

The SFV has the advantage that genomic replication occurs in the cytoplasm, where the viral replicase transcribes and caps the subgenomes for production of the structural proteins. Moreover, the genome of the SFV has several features that make it an ideal choice for a gene transfer vehicle for vaccination purposes: (1) it has a broad host range (2) it has an RNA genome of positive polarity that functions like mRNA, (3) its RNA is efficiently replicated in the cytoplasm, (4) infection leads to cell death, resulting in cross priming of the encoded antigen, and (5) it is safe to work with. SFV-based expression vectors are based on a full-length cDNA clone from which the 26S structural genes are deleted and are replaced by the heterologous insert.

Liljeström and coworkers developed a vector system that allows for efficient expression of foreign coding sequences as part of the SFV RNA replicon. A high biosafety level is obtained by separating the replicase and structural genes of the viral genome. Thus, recombinant virus particles can be produced that infect cells only once. These SFV replicons particles are extremely powerful as vaccines. As the antigens inserted in the vector are produced to high levels internally in the cell, this results in appropriate antigen processing and presentation. Furthermore, replication of the virus results in apoptosis and SFV double-stranded RNA (dsRNA) intermediates. Both are detected as "danger signals" providing an adjuvant effect to the vector encoded antigen (Quetglas et al, Virus Research 2010). This leads to the induction of type I interferons, which are the main cytokines involved in innate immune responses against viral infections, and specific activation of TLR3, 7 and 8. All together, these properties of SFV contribute to the induction of a strong T cell response against the expressed antigen.

SFV-based vaccines are known in the art. For example, the efficacy of rSFV expressing viral antigens has been evaluated in immunization studies in mice, guinea-pigs, monkeys and even in chicks. Examples of antigens extensively studied are the nucleoprotein and haemagglutinin of influenza virus (Berglund P., et al., Vaccine 1999), Human Papilloma virus (HPV) E6 and E7 protein (Daemen T., et al., Gene Therapy 2002) , and Hepatitis C virus ( Ip P.P., et al., Molecular Therapy 2014).

EP1370666B1 relates to an SFV-based system comprising nucleic acid of a human papilloma virus (HPV) origin. Infection of genital epithelial cells with high risk HPV types, in particular type 16 and 18, is closely associated with the development of cervical carcinoma. Immunization of mice with SFV particles encoding a fusion protein of HPV16 E6 and E7 resulted in a HPV-specific CTL response leading to the complete eradication of established TC-1 tumors (an HPV transfected murine cell line). Furthermore, tumor rechallenge studies demonstrated long-term protection. When previously immunized mice were rechallenged with tumor cells after three or six months, the mice were still protected from tumor outgrowth without another round of immunization and high levels of CTL activity could be measured even up to 11 months after immunization.

In view of the above, the potential and further development of SFV-based vaccines in a clinical setting looks promising. However, for commercial applications a reliable method for large scale purification of SFV replicon particles is required. Purification methods at a laboratory scale used in most scientific studies, e.g. using density gradient centrifugation of sucrose, cesium chloride, and the like, typically do not allow for scaling up in a cost- and time-effective manner.

The present inventors therefore sought to provide a novel purification process that allows for the large scale manufacture of a composition comprising highly purified SFV replicon particles at a high viral titer.

It was surprisingly found that this goal could be met by a two-step chromatographic procedure comprising subjecting a pretreated harvest of viral particles to a strong anion exchange column, followed by a second chromatography step using a cation exchange resin, and wherein a zwitterionic buffer is used during the chromatographic procedure. The fraction thus obtained is stabilized to allow for long term (> 2 years) storage.

Accordingly, in one embodiment the invention provides a method for providing purified viral particles of Semliki Forest Virus (SFV), comprising the steps of
i) providing a preparation of crude SFV replicon particles;
ii) subjecting said preparation to an endonuclease treatment under conditions allowing for degradation of host cell DNA and RNA;
iii) bringing said endonuclease-treated preparation with a zwitterionic buffer solution to a conductivity in the range of 4.5 - 5.0 mS/cm;
iv) contacting the preparation obtained in step (iii) with a strong anion exchange resin under conditions and for a time sufficient to bind to said resin, followed by washing the resin with a zwitterionic buffer solution to remove the portion of the preparation which does not bind to said anion exchange resin from said anion exchange resin;
v) eluting the bound SFV replicon particles from said anion exchange resin;
vi) bringing the eluted SFV particles to a conductivity in the range of 7.5-8.2 mS/cm;
vii) contacting the preparation obtained in step (vi) with a strong cation exchange resin under conditions and for a time sufficient to bind to said resin, followed by washing the resin to remove the portion of the preparation which does not bind to said cation exchange resin from said cation exchange resin;
viii) eluting the bound SFV replicon particles from said cation exchange resin with a zwitterionic buffer solution and collecting at least one fraction containing purified SFV replicon particles; and
ix) stabilizing the at least one purified fraction by adding human serum albumin (HSA) to a final concentration in the range of 0.5 - 2 w/v%.

As used herein, the term 'zwitterionic buffer solution" refers to an aqueous solution comprising one or more zwitterion buffer salts. Zwitterionic buffers were first described by Good et al., (Biochemistry (1966) 5:467). In one embodiment, the zwitterionic buffer is an amino-alkane acid buffer. In another embodiment, the zwitterionic buffer is an aminoalkyl group sulfonate buffer. The zwitterionic buffer preferably has a pKa value at 20°C in the range 6.0 to 10.5.

Illustrative zwitterionic buffers include 2-(N-morpholino) ethyl sulfonic acid (MES), piperazine-N, N '-two (2-ethanesulfonic acid) (PIPES), N-(2-ethanamide)-2-aminoethyl sulfonic acid (ACES), (4-(2-hydroxyethyl)-1-piperazine ethanesulfonic acid) (HEPES), N-(tri-(methylol) methyl) glycine (N-tri-(methylol) methylglycine), N, N-bis-(2-hydroxyethyl) glycine; (N, N-bis-(hydroxyethyl) glycine), MOPS (MOPS), N-cyclohexyl-Homotaurine (CAPS), N, N-bis-(2-hydroxyethyl)-2-aminoethyl sulfonic acid (BES), N-(hydroxyethyl) piperazine-N'-2-hydroxy-propanesulfonic acid (HEPPSO) and TAPSO (3-[N-tris(hydroxymethyl)methylamino]-2-hydroxy propanesulfonic acid).

In a preferred embodiment, the zwitterionic buffer is PIPES. In another preferred embodiment, the zwitterionic buffer is MOPS. In a most preferred embodiment, the zwitterionic buffer is HEPES.

Zwitterionic buffers may be used individually or in combination such that the final concentration is between about 10 mM and about 100 mM, preferably 30-70 mM, preferably about 50 mM.In a specific aspect, the buffer solution used in at least one, preferably all, of the steps is a HEPES buffer. For instance, a 40-60 mM HEPES buffer pH 7.0 ± 0.5, preferably pH 7.0 ± 0.3, is used in all steps.

In one embodiment, the zwitterionic elution buffer in step vii) comprises about 250-350 mM NaCl buffer and/or the elution buffer in step viii) comprises 500-700 mM NaCl. In one embodiment, the elution buffer in step vii) is 50 mM HEPES pH 7.0, 0.3 M NaCl buffer and/or the elution buffer step viii) is 50 mM HEPES with 0.6 M NaCl.

A chromatographic purification method provided herein allows for the purification of large volumes of crude viral harvests. The viral particles of interest can interact with the chromatic medium by a simple adsorption/desorption mechanism, and large volumes of sample can be processed in a single load. Contaminants that do not have affinity for the adsorbent pass through the column. The replicon particles can then be eluted in a relatively concentrated form.

Methods for purifying viral particles, including alphavirus particles, using one or more chromatographic steps are known in the art. WO95/07994, relating to composition and methods for utilizing recombinant alphavirus vectors, teaches that crude recombinant virus may be purified by ion exchange column chromatography. For example, crude recombinant virus may be clarified by first passing it through a filter, followed by loading the filtrate onto a column containing a highly sulfonated cellulose matrix. The recombinant virus may then be eluted from the column in purified form by using a high salt buffer, and the high salt buffer exchanged for a more desirable buffer by passing the eluate over a molecular exclusion column. WO95/07994 also discloses the use of HSA as structural additive to prevent viral aggregation.

US2002/0015945 discloses methods of purification that subject alphavirus replicon particle preparations to one or more steps of chromatographic purification, such as using an ion exchange resin. It is generally taught that a method of purification for alphavirus replicon particles may comprise at least two (sequential) chromatographic purification steps, selected from the group consisting of ion exchange chromatography, size exclusion chromatography, hydrophobic interaction chromatography, and affinity chromatography. According to US2002/0015945, a preferred purification process uses a first step of ion exchange chromatography and a second step of size exclusion chromatography. All examples relate to purification of replicon particles of Sindbis (SIN) virus.

WO2004/055167 relates to methods for producing alphavirus replicon particles in high yield. Replicon RNAs are electroporated into permissive cells, where the cells are at a relatively high density, together with at least one helper nucleic acid providing the necessary functions for packaging. After a growth period in appropriate medium, alphavirus replicon particles are harvested using a salt wash. After dilution, if necessary, the particles can be purified by a suitable chromatographic technique. WO2004/055167 specifically discloses a process comprising hydrophobic interaction chromatography (HIC) followed by anion exchange chromatography.

EP2848692 relates to the purification of alphavirus replicons. It generally suggests first treating the replicons with an endonuclease, followed by purification on a strong anion exchange resin, followed by purification on a strong cation exchange resin. HSA may be added up to a preferred concentration of 0.1 w/v%. The only exemplified ion exchange purification method in EP2848692 concerns the purification of inactivated pseudoinfectious virus (PIV) replicons by cation exchange chromatography using a sodium phosphate buffer solution.

However, a method herein disclosed requiring the stepwise application of SFV viral particles to an anion exchange chromatography column, followed by cation exchange chromatography wherein a zwitterionic buffer solution is used in both chromatographic steps is not taught or suggested in the art. Also, the art is silent about the final formulation of purified virus particles in a zwitterionic buffer comprising 0.5-2 w/v% HSA.

Step i) of a method provided herein comprises providing a preparation of crude SFV replicon particles. Typically, the viral replicon particles are produced by host cells and harvested from the supernatant or growth medium wherein host cells are cultured. These SFV replicon particles according to the present invention may be produced using a variety of published methods for alphavirus replicon particle production. Such methods include, for example, transient packaging approaches, such as the co-transfection of *in vitro* transcribed replicon and one or more defective helper RNA(s) (Liljeström, Bio/Technology 9:1356-1361, 1991 ; Bredenbeek et al., J. Virol. (57:6439-6446, 1993; Frolov et al., J. Virol. 7i:2819-2S29, 1997; Pushko et al., Virology 239:389-401, 1997; U.S. Patents 5,789,245) or plasmid DNA-based replicon and defective helper constructs (Dubensky et al., J. Virol. 70:508-519, 1996), as well as introduction of alphavirus replicons into stable packaging cell lines (PCL) (U.S. Patents 5,759,245, 5,842,723, 6,015,694; WO 97/38087, WO 99/18226, WO 00/61772, WO 00/39318, and WO 01/92552).

In a preferred embodiment, a method of the invention utilizes stable SFV packaging cell lines for replicon particle production. The packaging cell lines may be transfected with *in vitro* transcribed replicon RNA, transfected with plasmid DNA-based replicon, or infected with a seed stock of replicon particles, and then incubated under conditions and for a time sufficient to produce high titer packaged replicon particles in the culture supernatant. It typically comprises producing a host cell that is modified to produce viral particles by introducing an SFV replicon nucleic acid (or an SFV nucleic acid) into a host cell, the replicon nucleic acid containing at least an SFV packaging signal and at least one coding sequence for a gene (s) of interest. The SFV nucleic acid can be a wild-type virus, a recombinant virus which comprises a nucleotide sequence of interest as described above, or it can be an attenuated virus useful for producing an immunogenic response in the host. The modified host cell is then cultured in a medium under conditions allowing expression of the structural proteins and replication of the SFV replicon nucleic acid, and then packaging of the SFV replicon nucleic acid to form SFV replicon particles.

In a specific aspect, the packaging cell line contains all components, including the replicon RNA, under an inducible promoter. This allows for a producer cell that can be cultured and that will only produce virus upon induction with a specific inducing agent.

Suitable host cells include cultured mammalian and cultured insect cells. Vero cells, baby hamster kidney (BHK) cells, chicken embryo fibroblast (CEF) cells, DF-1,293, 293T, Chinese Hamster Ovary (CHO) cells are especially useful. In a specific aspect, Vero cells are used.

Modified host cells can then be placed in culture vessels containing growth medium. When using adherent host cells, the vessel contains a volume of growth medium that is sufficient to allow cell growth, replicon RNA replication, packaging, protein expression and viral replicon particle production, and sufficient surface area for those cells to attach and grow. The surface may be vessel, flask or plate walls, or hollow fibers, beads or any other carrier appropriate for attached cell growth can be placed within the vessel. For example, Vero cells or other cells grown on microcarriers (beads or discs) can be successfully electroporated on the carriers and cultured to produce replicon particles at levels comparable to those achieved with unattached cells.

Large-scale production of SFV replicon particles may be performed using a bioreactor. For example, the bioreactor is an external component bioreactor, which is an integrated modular bioreactor system for the mass culture, growth, and process control of substrate attached cells. The attachment and propagation of cells occurs in a vessel or chamber with tissue culture treated surfaces, and the cells are provided with fresh media for increased cell productivity. Alternatively, cells can be grown in suspension culture in bioreactors. Monitoring and adjustments are performed for such parameters as gases, temperature, pH, glucose, etc., and crude vector is harvested using a perfusion pump.

After the cells have grown and produced particles, typically from about 12 to about 48 hours, the particles are harvested by collection of the growth medium (supernatant).

In step ii), the preparation is subjected to an endonuclease treatment under conditions allowing for degradation of host cell DNA and RNA. This method step is also known in the art. Typically, an endonuclease (e.g., Benzonase, Sigma #E8263) is added to the preparation of replicon particles to digest exogenous nucleic acid. The enzyme reaction can be terminated by dilution, e.g. in a 1:1 fashion, with buffer containing a chelating agent such as EDTA. Further, the preparation may be concentrated prior to purification using one of any widely known methods (e.g., tangential flow filtration).

Following endonuclease treatment, the preparation is brought to a conductivity up to about 5.5 mS/cm using (dilution with) a suitable zwitterionic buffer solution. This adjustment was found to be important for the recovery of viral particles from the anion exchange chromatography step. According to the invention, the preparation to be loaded onto the first column has a conductivity in the range of 4.5 - 5.0 mS/cm.

Step iv) of a method provided herein comprises the first actual chromatographic purification process. Briefly, the process comprises conventional ion exchange chromatographic principles, including column equilibration, sample loading, column washing, and elution.

Therein, the preparation obtained in step (iii) is contacted with a strong anion exchange resin under conditions and for a time sufficient to bind to said resin. Anion-exchange chromatography is a well-established process that separates substances based on their charges using an ion-exchange resin containing positively charged groups, such as diethyl-aminoethyl groups (DEAE). In solution, the resin is coated with positively charged counter-ions (cations). Anion exchange resins will bind to negatively charged molecules, displacing the counter-ion. Anion exchange chromatography is commonly used to purify proteins, amino acids, sugars/carbohydrates and other acidic substances with a negative charge at higher pH levels. The tightness of the binding between the substance and the resin is based on the strength of the negative charge of the substance. Strong anion exchange resins for use in the present invention are known in the art. Preferred resins include those the functional group is of the quaternary amines (QA) type that rely on quaternary amines for binding acidic groups on materials passed over them.

In a specific aspect, a method of the invention employs in one or both chromatographic steps a monolithic column. Monoliths are unique from other forms of chromatography media for several reasons. Monolith architecture consists of highly interconnected convective channels that are distributed throughout the entire bed. The large channels are easily available for purification even for large biomolecules. This unique architecture also creates a void-less space, thus significantly reducing shear and product loss. In monoliths, the mass transport is also exclusively convective and laminar which means that all solutes flow with the current regardless of size and require only a few seconds of residence time. These features make monoliths well suited for the purification of virus particles. Accordingly, in a specific embodiment a monolith column comprising a QA resin is used. Such columns are commercially available e.g. from BIA Separations ( Mirce, Slovenia). Very good results can be obtained with a QA-type monolithic column having a highly porous methacrylate based monolithic material, preferably having an average pore radius in the range of about 600 to 750 nm. For example, a CIM^{®} QA-8 cGMP tube monolithic column is used.

After the complete sample is loaded onto the column, the resin is washed with a zwitterionic buffer solution to remove the portion of the preparation which does not bind to the anion exchange resin. Typically, the column is washed with loading buffer until no protein is detected in the flow through. This may require ≥ 5 column volumes.

Following washing, the bound SFV replicon particles are eluted from the anion exchange resin using an appropriate salt-containing zwitterionic buffer. Generally speaking, particles can be eluted either in a linear, gradient elution or using a step isocratic elution. A gradient elution may be used to optimize elution conditions. Once the elution profile of the protein of interest has been established and it is known at what ionic strength or pH the viral particles elute, a step elution can be used to speed the purification process. Useful (step) elution buffers include those containing 0.25 to 0.4 M NaCl. In one embodiment, a 0.3 M NaCl buffer solution is used. The anion exchange resin is suitably regenerated by washing with a high salt buffer, e.g. a buffer containing at least 0.5 M NaCl.

In step vi), the anion exchange purified SFV particles are brought to a conductivity in the range of 7.5 to 8.2 mS/cm. This can be done in any suitable manner, such as buffer dilution or tangential flow filtration (TFF) using an appropriate buffer.

Step vii) entails the second chromatographic purification step, wherein the preparation obtained in step (vi) is contacted with a strong cation exchange resin, under conditions and for a time sufficient to bind to said resin, followed by washing the resin to remove the portion of the preparation which does not bind to said cation exchange resin from said cation exchange resin. Cation exchange chromatography uses a negatively charged ion exchange resin with an affinity for molecules having net positive surface charges. To recover the analyte, the resin is then washed with a solvent neutralizing this ionic interaction. In one embodiment, the replicon particles are bound to the cation exchange resin followed by one or more washes with buffer containing a salt (e.g., 250 mM or less NaCl). Strong cation exchange resin (SCX) resins for use in the present invention are known in the art. Exemplary cationic exchange materials include a sulphopropyl cation exchanger, a carboxymethyl cation exchanger, a sulfonic acid exchanger, a methyl sulfonate cation exchanger, and an SO3- exchanger. The SCX stationary phase usually contains aliphatic sulfonic acid groups that are negatively charged in aqueous solution, therefore tightly binding any strongly basic analytes. Accordingly, in a preferred embodiment the second chromatographic step is performed using a resin of the sulfonyl/SO3 type.

Subsequently, in step viii) the bound SFV replicon particles are eluted from the cation exchange resin with a zwitterionic buffer solution, and at least one fraction containing purified SFV replicon particles is collected. In one aspect, replicon particles are eluted from the column in purified form using a buffer with increased salt concentration. In preferred embodiments, the salt concentration is a least 350 mM, 400 mM, 450 mM, 500 mM or even higher. In one aspect, viral particles are eluted with 600 mM NaCl. Elution may be monitored preferably by a spectrophotometer at 280 nm, but also by replicon (infectious) titer assay or genomic titer assay using quantitative reversed transcriptase-PCR, transfer of expression (TOE) assay, or protein gel analysis with subsequent Coomassie staining or Western blotting.

In step ix), the at least one purified fraction, or two or more pooled fractions, is/are stabilized by adding human serum albumin (HSA) to a final concentration in the range of 0.5 - 2 w/v%. A very good storage stability was observed with a final HSA concentration of about 1 w/v%.

If desired, the virus particle concentration in the stabilized preparation may be reduced or increased and/or the salt concentration can be adjusted. This can be achieved using tangential flow filtration procedures known per se in the art. Exemplary preparation for clinical applications may contain infectious viral particles in the concentration range of about 2.5x10exp5/mL to about 1x10exp10/mL, for example 2.5x10exp5/mL to 5 x 10 exp8/mL.

Also, the stabilized preparation can be subjected to a filter sterilization step.

A method of the invention may furthermore comprise performing, in between steps iii) and iv), a filtration or tangential flow filtration (TFF) step. An additional depth filtration prior to the first chromatography step allows for removing contaminating (host cell) protein and DNA. TFF allows for removal of small sized impurities and/or zwitterionic buffer exchange by cycles of concentration and diafiltration through the pores of the filter. Herewith, TFF is advantageously used to further concentrate the virus and to bring the virus to the appropriate conductivity prior to loading on the column.

As indicated herein above, the SFV replicon nucleic acid can comprise a nucleotide sequence of interest to be expressed in the host cell. The nucleotide sequence can encode an immunogenic polypeptide, a cytokine or a therapeutic protein, among others, or the nucleotide sequence can be transcribed in the host cell to produce a functional RNA, such as a ribozyme, an interfering or an antisense molecule. In one embodiment, the SFV particles comprise a vector system comprising a nucleic acid molecule encoding a pathogen-derived antigen or a cancer-derived antigen. For example, the antigen is derived from a virus, bacterium, fungus or parasite.

An "antigen" refers to a molecule containing one or more epitopes (either linear, conformational or both) that will stimulate a host's immune system to make a humoral and/or cellular antigen-specific response. The term is used interchangeably with the term, "immunogen," Normally, an epitope will include between about 3-15, generally about 5-15 amino acids. A B-cell epitope is normally about 5 amino acids but can be as small as 3-4 amino acids. A T-cell epitope, such as a CTL epitope, will include at least about 7-9 amino acids, and a helper T-cell epitope at least about 12-20 amino acids. Normally, an epitope will include between about 7 and 15 amino acids, such as, 9, 10, 12 or 15 amino acids. The term "antigen" denotes both subunit antigens, (i.e., antigens which are separate and discrete from a whole organism with which the antigen is associated in nature), as well as, killed, attenuated or inactivated bacteria, viruses, fungi, parasites or other microbes as well as tumor antigens, including extracellular domains of cell surface receptors and intracellular portions that may contain T-cell epitopes. Antibodies such as anti-idiotype antibodies, or fragments thereof, and synthetic peptide mimotopes, which can mimic an antigen or antigenic determinant, are also captured under the definition of antigen as used herein. Similarly, an oligonucleotide or polynucleotide that expresses an antigen or antigenic determinant in vivo, such as in gene therapy and DNA immunization applications, is also included in the definition of antigen herein.

For purposes of the present invention, antigens can be derived from any of several known viruses, bacteria, parasites and fungi, as described more fully below. The term also intends any other antigen to which an immune response is desired. Furthermore, for purposes of the present invention, an "antigen" refers to a protein that includes modifications, such as deletions, additions and substitutions (generally conservative in nature), to the native sequence, so long as the protein maintains the ability to elicit an immunological response, as defined herein. These modifications may be deliberate, as through site-directed mutagenesis, or may be accidental, such as through mutations of hosts that produce the antigens.

In a specific aspect, the antigen is a viral antigen of human papilloma virus (HPV). For example, the nucleic acid molecule encodes at least one antigenic polypeptide (fragment) of HPV. HPV antigen proteins include any immunogenic antigen protein or peptide capable of eliciting an immune response against HPV virus. Generally, immunogenic proteins of interest include HPV antigens (e.g., E6 protein, E7 protein, etc.), an immunogenic portion thereof, and/or an immunogenic variant thereof. HPV antigens for use in accordance with the present invention may include full-length HPV proteins (e.g., E6, E7, etc.), or fragments of such proteins, where such fragments retain immunological activity, and/or fusion proteins comprising such full-length HPV proteins or fragments. See for example EP1370666 B1.

In one embodiment, the HPV antigen fragment is of a protein E6 or a protein E7 origin, preferably wherein said polypeptide fragment comprises an antigenic polypeptide fragment of the protein E6 and an antigenic polypeptide fragment of the protein E7. The invention may also comprise the use of an SFV vector system comprising a nucleic acid encoding at least one antigenic polypeptide fragment of HPV, wherein said antigenic polypeptide fragment comprises one antigenic polypeptide fragment of E6 and one antigenic polypeptide fragment of E7. Antigenic polypeptide fragment of HPV herein refers to tumour antigens E6 and E7 that can bind to the cellular tumour suppressor products pRB and P53. Preferably, nucleic acid encoding E6 and E7 or fragments thereof are fused in frame. Alternatively, the SFV vector system may comprise a nucleic acid wherein said nucleic acid encodes at least one antigenic polypeptide fragment of protein E6 and/or protein E7 of a HPV wherein said antigenic polypeptide fragment is at least partially been deprived of the capacity to bind to pRb and/or P53 protein. In a preferred embodiment, a method of the invention employs an SFV replicon particle, also designated a replication deficient recombinant Semliki Forest virus (rSFV) vector, expressing a fusion protein of HPV E6,7.

The invention furthermore relates to the product obtainable by the purification methods provided herein. Disclosed is a stabilized preparation of purified SFV replicon particles obtainable by or obtained by a method according to the invention. This preparation contains highly purified SFV replicon particles in a zwitterionic buffer system comprising 0.5-2 w/v%, preferably about 1 w/v %, HSA. It is suitable for use in human and veterinary medicine, and has a long storage stability, e.g. up to at least 18 months, preferably up to at least 24 months, more preferably up to at least 36 months, storage at -60°C or lower.

As used herein, the storage stability is reflected by a viral titer that does not change significantly during the storage period, i.e. within the variability for the viral titer assay used, which is typically about ±0.3 log10. In one embodiment, the stabilized formulation has a viral titer loss of less than 0.2 log10.

As used herein, purified shall mean an SFV replicon particle preparation that is essentially free (deprived) from detectable non-alphavirus proteins. Detection of non-alphavirus proteins is suitably determined by gel electrophoresis using a sample size of between approximately 10exp8 to 10exp9 replicon particles. Gel electrophoresis methods include polyacrylamide gel electrophoresis (PAGE), disc electrophoresis and SDS-PAGE, followed by standard Coomassie staining. More specifically, "purified" shall mean SFV particle preparations subjected to multistep chromatography purification procedures as disclosed herein.

In one embodiment, the preparation contains viral particles comprised in a HEPES buffer. Preferably, the buffer is 40-60 mM HEPES pH 7.0 ± 0.3. A preparation of the invention is further characterized by a clear or slightly opalescent appearance. The preparation typically has an osmolality in a range between 250-600 mOsmol/kg, e.g. of 390 +/- 40 mOsmol/kg . The viral titer can vary but typically ranges from 2.5x10exp5/mL to about 1x10exp10/mL.

The invention also provides a pharmaceutical composition comprising a stabilized preparation of purified SFV replicon particles and a pharmaceutically acceptable carrier, vehicle or diluent, wherein the particles show less than 0.3log10 change in viral titer upon ≥ 18 months storage at - 60 °C or lower, and wherein the composition is formulated with a HEPES buffer system at pH 7.0 ± 0.3, 200 - 250 mM NaCl, and 1-2 w/v% HSA in water for injection (WFI). For example, the invention provides a pharmaceutical composition comprising a stabilized preparation of purified SFV replicon particles in HEPES 19 mM, NaCl 227 mM, and HSA 1% in WFI at pH 7.0.

Pharmaceutical compositions, such as vaccines or other immunogenic compositions, of the present invention comprise an immunogenic amount of the infectious, propagation defective SFV replicon particles or live, attenuated particles in combination with a pharmaceutically acceptable carrier. An "immunogenic amount" is an amount of the infectious SFV particles which is sufficient to evoke an immune response in the subject to which the pharmaceutical formulation is administered. An amount of from about 10exp4 to about 10exp10 especially 10exp6 to 10exp8, infectious units, or replicon particles per dose is believed suitable, depending e.g. upon the age, route of injection and species of the subject being treated.

One or more immuno-potentiator molecules, such as chemokines and/or cytokines can be incorporated in the immunogenic compositions comprising the SFV replicon particles prepared as described herein. Alternatively, the immunogenic compositions can comprise SFV replicon particles which direct the expression of one or more chemokines and/or cytokines in the subject to which the composition is administered. Exemplary chemokines and/or cytokines include interleukin-4, interleukin-12, gamma-interferon, granulocyte macrophage colony stimulating factor, and FLT-3 ligand. It is understood that the choice of cytokine and/or chemokine may vary according to the neoplasia, parasite or pathogen which is targeted for an immune response.

In a specific embodiment, the immunogenic composition comprises SFV replicon particles which direct the expression of a HPV antigen. As is exemplified herein below, the invention provides SFV particles comprising a recombinant, attenuated, replication-incompetent form of the SFV vector encoding the viral oncoproteins E6 and E7 derived from HPV, with potential immunomodulating and antineoplastic activities. This HPV E6/E7-encoding SFV vaccine is suitably used, e.g. upon intramuscular administration, to induce expression of the E6/E7 proteins and to stimulate both the innate and the adaptive immune system, resulting in a potent cytotoxic T-lymphocyte (CTL) response against and lysis of tumor cells expressing HPV E6 and E7. Oncoproteins E6 and E7 play a key role in the development of cervical intraepithelial neoplasia (CIN), cervical carcinoma, as well as other anogenital cancers and head and neck cancers.

Still further, the invention provides a pharmaceutical composition according to the invention for use in a method for vaccinating a subject, comprising administering to the subject an effective amount of said pharmaceutical composition. Administration may be by any suitable means, such as intraperitoneal, intramuscular, intradermal, intranasal, intravaginal, intrarectal, intratumoral, subcutaneous or intravenous administration. In a specific aspect, the pharmaceutical composition comprises SFV particles comprising a vector encoding the viral oncoproteins E6 and E7. In a preferred embodiment, the composition is administered by injection in at least a left and right limb, more preferably in at least the right and left (upper) leg, of said subject.
Accordingly, also disclosed but not part of the invention is a method of treating or preventing a disease caused by HPV, in particular cervical cancer, comprising administering purified SFV replicon particles comprising nucleic acid derived from human papilloma virus (HPV) to a subject. HPV, commonly known as the virus that causes genital warts and cervical cancer in women, is increasingly being recognized now as a cause of infections that colonize the back of the mouth (throat or oropharynx), including the tongue base and tonsils, and potentially a cause of cancer of the head and neck. Accordingly, the method can comprises treating or preventing HPV infections of the mouth, in particular tonsillar, throat or oropharyngeal HPV infection.

### LEGEND TO THE FIGURES

Fig. 1 is a schematic representation of the production of SFV particles in Vero cells using three RNA transcripts, one encoding the replicase and the protein of interest, one encoding the SFV capsid protein, and one encoding the SFV spike proteins.
Fig. 2 presents the maps of the three plasmids pSFVeE6,7 encoding the replicase and E6,7 fusion protein (A), pSFV-helper-C-S219A encoding the capsid protein (B) and pSFV-helper-S2 encoding the spike proteins (C).
Fig. 3 presents the chromatograms of the CIM^{®}-QA-8cGMP column (A) and CIM^{®}-SO3-8cGMP column (B), showing the elution of SFV replicons from these columns in the purification run of SFV replicon particle batch number 16-A26-12. Curves represent the absorbance (Left axis in mAU) at 280 nm and the conductivity (right axis in mS/cm) of each run
Fig. 4 provides the results of SFV replicon particle purification on single staged CIM QA discs and CIM SO3 discs. Recovery in terms of % virus recovery (infectious viral particles), total (host cell) protein and total host cell DNA compared to viral load before purification are plotted.

### EXPERIMENTAL SECTION

### EXAMPLE 1: Preparation of host cells containing SFV replicon particles, virus harvest and nuclease treatment.

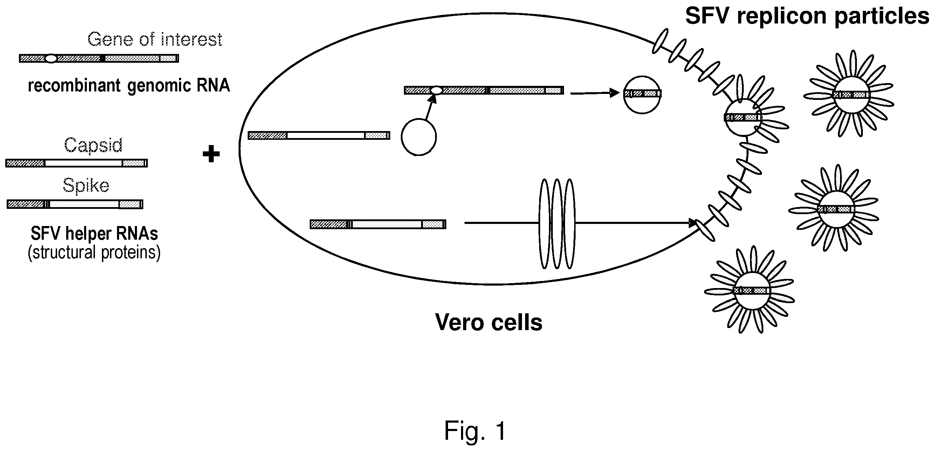

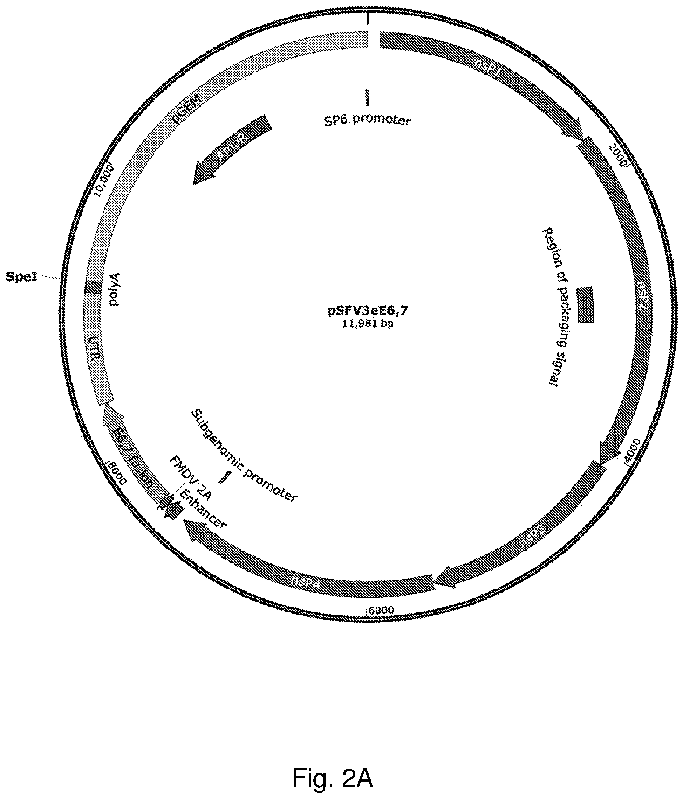

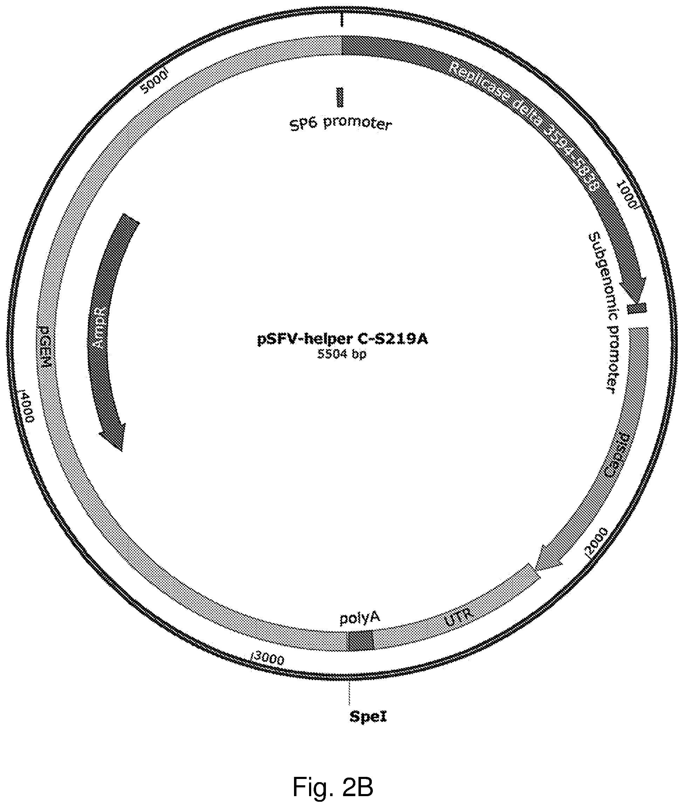

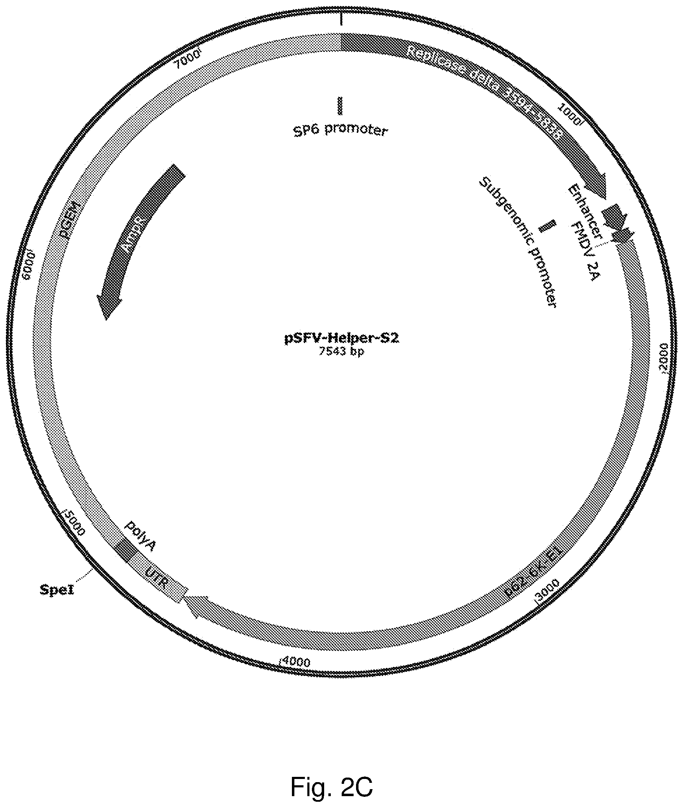

In order to produce SFV replicon particles, Vero cells (kidney cells from an African green monkey) are electroporated with three transcripts (Fig. 1). These transcripts are derived from three plasmids i.e. pSFVeE6,7 encoding the replicase and E6,7 fusion protein, pSFV-helper-C-S219A encoding the capsid protein and pSFV-helper-S2 encoding the spike proteins. DNA of each individual plasmid was linearized via a digestion reaction using the BcuI (=SpeI) restriction enzyme, which has one recognition site in each plasmid (Fig. 2). The DNA was incubated with BcuI at 36-38 °C for 2 hours (+/- 10 minutes). The restriction reaction was stopped by adding a mixture of EDTA, ammonium acetate and ethanol. DNA was precipitated by centrifugation at 20,000 g and the resulting pellet is dried at room temperature. The DNA pellet was dissolved in nuclease-free water before storage.
The DNA was analyzed for concentration, absence of circular DNA by gel electrophoresis and absence of proteins by determination of the ratio OD 260/280nm. RNA was then synthesized via *in vitro* transcription using SP6 RNA polymerase, which recognizes the SP6 promoter in each of the linearized DNA constructs. Linearized DNA of the three constructs was incubated with SP6 polymerase and nucleotides at 37 °C for 2 hours ± 10 minutes. Next, the RNA was incubated with TURBO DNase to remove template DNA. The purified RNA was recovered by lithium chloride precipitation and centrifugation (20 minutes, 20,000 g, 4 °C) and the resulting pellet dissolved in nuclease-free water. The purified RNA was tested for concentration, purity and endotoxins.

SFV viral replicon particles were produced in Vero cells after electroporation of the three RNA transcripts. Vero cells were cultured in culture medium (Medium 199 w/o phenol red) with 10 % FBS and 2 mM L-glutamine (size 175 cm2) at 36-38°C and 4-6 % CO₂ to a cell confluency of 80-90 %. At every cell passage, a single cell suspension was generated by recombinant trypsin (Tryple Select) treatment to dislodge the cells from the tissue flasks. Cells were passaged based on cell confluency and a passaging frequency of twice a week. Vero cells from subconfluent flasks were detached with Tryple Select and resuspended in Biorad electroporation buffer in a concentration of 20x10⁶/ml. Cells were transferred to Gene pulser cuvettes and electroporated with the RNA transcripts using a Gene pulser Xcell electroporation unit. Each electroporation was performed with 16x10⁶ cells and 11.3 µg helper C, 15.4 µg helper S and 36.0 µg RNA E6, 7 RNA.

The following electroporation conditions were applied: 195V, 5.0 msec pulse length, 4 pulses, pulse interval 0.1sec. The transfected Vero cells were seeded back at a cell density of 20x10⁶ cells/10 mL into 75 cm² tissue culture flasks and incubated for virus propagation for up to two days at 37 °C and 5% CO₂ in culture medium 199 with Earle's BSS containing 5% FBS and 2 mM L-glutamine. Viral particles were harvested after 24 and 48 hours by collection of the supernatant and centrifugation to remove Vero cells/cell debris. Viral titer and bioburden were determined for each harvest. Individual harvests were stored at ≤-60 °C until purification. Virus titers of individual harvests typically range between 2x10⁷-1.3x10⁹ infectious particles/mL.

Individual crude SFV harvests were pooled for purification. Virus purification starts with thawing of the virus harvest(s) at 37 °C. The thawed harvests were pooled in a sterile glass container. As shown in Table 1 herein below, titers of pooled harvests are typically between 4x10⁷-2x10⁸ infectious particles/mL. The pooled harvest was treated with benzonase (50 U/mL) in 2 mM magnesium chloride for 30 minutes at 37 °C, in order to degrade all forms of host cell nucleic acid (DNA and RNA). The benzonase reaction was terminated by 1:1 v/v dilution with 10 mM EDTA in 50 mM HEPES buffer pH 7.0. The benzonase-treated material was then prepared for the first purification step by diluting with 50mM HEPES buffer pH 7.0-7.5 to a conductivity of 4.5-5.0 mS/cm.

### EXAMPLE 2: Two-step Chromatographic Purification

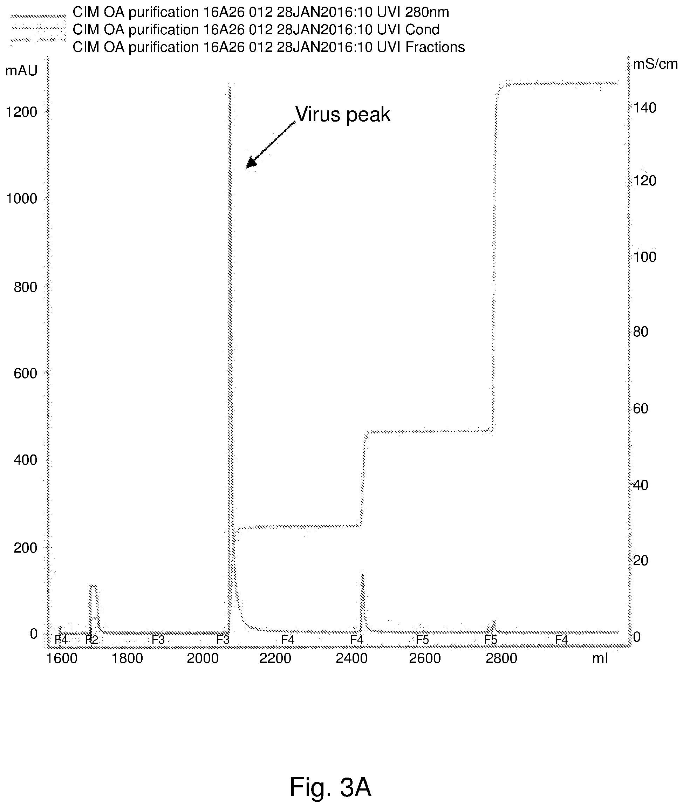

For the purification of the benzonase-treated preparation of SFV particles, an ÄKTA pilot chromatography system was used. In the first step, the diluted harvest pool was loaded on a solid state ceramic strong anion exchange column (CIM^{®}-QA-8cGMP tube monolithic column from Bia Separations), which was used in a binding mode. After loading, the column was washed with a 50 mM HEPES buffer pH 7.0-7.5. Subsequently the virus particles were eluted using a 50mM HEPES buffer 7.0-7.5 containing 0.3 M NaCl. An example of a chromatogram showing elution of the virus from the CIM^{®}-QA-8cGMP column is shown in figure 3A.

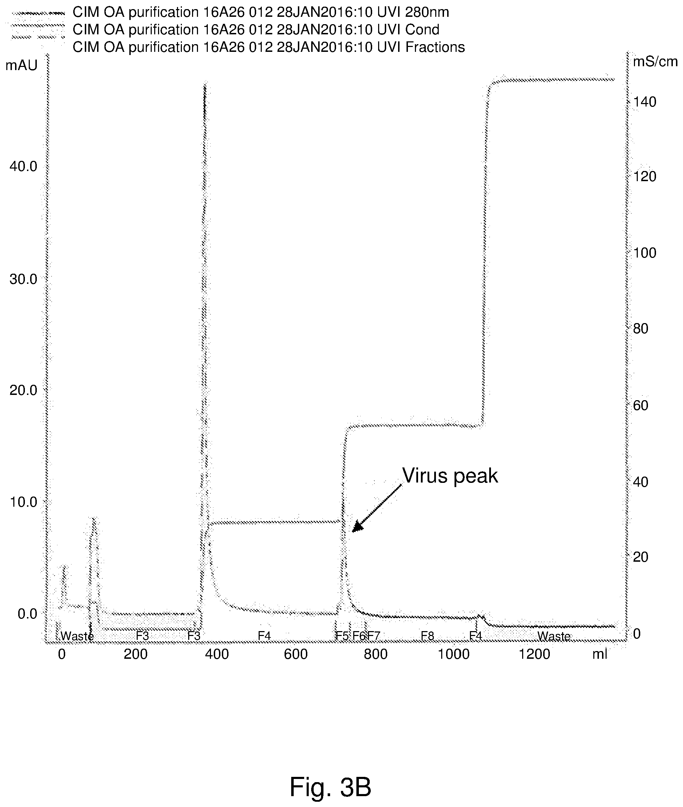

In a second step, the anion exchange purified virus was subjected to cation exchange chromatography using a CIM^{®}-SO3-8cGMP tube monolithic column from Bia Separations, again in binding mode. Before loading the virus on the column, the eluate obtained from the first chromatographic step was diluted with a 50 mM HEPES buffer pH 7.0-7.5 to a conductivity of 7.5-8.2 mS/cm. After loading, the column was washed with 50 mM HEPES buffer. Additional impurities were removed by elution with a 50 mM HEPES buffer containing 0.3 M NaCl. Subsequently, the viral particles were eluted with a 50mM HEPES buffer containing 0.6 M NaCl. The eluted virus was collected in sub-fractions. An example of a chromatogram showing the elution of the virus from the CIM^{®}-SO3-8cGMP column is shown in figure 3B.

Human Serum Albumin (HSA) was added to a final concentration of 1 w/v % to the purified virus fractions as a stabilizer, and the fractions were stored at ≤-60 °C for purity and quantity assessment prior to processing to the formulated Drug Substance.

The flowchart of Scheme 1 summarizes the individual steps performed in Examples 1 and 2.

Step no.
Process step
In process and
quality control
testing
1.
Thawing and pooling of
individual harvests
Viral titer,
mycoplasma,
bioburden, in vitro
and in vivo
adventitious virus,
size residual DNA
fragment
2.
Benzonase treatment
3.
Purification on anion
exchange column using
zwitterionic buffer
Viral titer,
conductivity, total
protein, host cell DNA
4.
Purification on cation
exchange column using
zwitterionic buffer
Viral titer,
conductivity, total
protein, Vero host cell
protein, host cell
DNA, total viral
particles
5.
Addition of HSA &
storage of purified
fractions
Viral titer, ratio total
particles/ infectious
particles
Scheme 1

### EXAMPLE 3: Characterization of the purified SFV replicon particles.

This example describes the characterization of products of the intermediate steps and the final product using a set of different assays (A through H). It demonstrates an appropriate removal of impurities, such as host cell DNA and protein, and recovery of infectious SFV replicon particles.

### A) Viral titer assay to detect infectious viral (SFV) particles and confirm identity.

The viral titer assay is used to determine the amount of infectious particles per milliliter (titer), as well as the identity of the SFV particles. The assay is performed by titration of viral particles by serial dilution on monolayers of BHK cells. Infection by SFV particles is determined by immunofluorescence using an antibody against SFV-nsp3 (replicase). This antibody was chosen because replicase is present in all cells infected with recombinant SFV. Positive staining therefore confirms the identity of the viral particles. Titers are calculated by counting positive cells and correction for the dilution factor.

### B) Agarose Gel Electrophoresis to determine the size of residual host cell DNA fragments

Agarose gel electrophoresis is performed using a 0.8 % agarose gel and Tris/Borate/EDTA running buffer. This method is used to determine the size of the residual DNA fragments in the crude pooled harvest. Biomolecules are separated by applying an electric field to move the negatively charged molecules through an agarose matrix. The separated DNA is visualized by using ethidium bromide stain and detection under UV light.

### C) Host cell DNA by Picogreen assay

The Picogreen assay allows to determine the level of host cell DNA in purified SFV virus samples. The assay makes use of the Quant-iT^{™} PicoGreen^{®} dsDNA reagent, which is a fluorescent nucleic acid stain for quantifying double-stranded DNA in solution. Fluorescence is detected using a fluorescence microplate reader with excitation wavelength ~480 nm and emission wavelength ~520 nm. DNA concentration is determined using a DNA standard curve.

### D) Host cell protein by ELISA

The residual Vero host cell protein levels were quantified using a Cygnus Technologies ELISA kit in which an affinity purified antibody directed against Vero cell proteins is attached to the solid phase (microtiter plate wells) and captured Vero proteins in SFV virus samples are detected by interaction with a horseradish peroxidase (HRP)-labeled anti-Vero antibody.

### E) MicroBCA assay to detect total protein including HSA

The MicroBCA assay is used to determine the level of total protein (i.e. host cell, virus protein in crude samples and HSA in purified samples) in SFV virus samples. The assay makes use of bicinchoninic acid (BCA) as the detection reagent for Cu+ 1, which is formed when Cu+2 is reduced by protein in an alkaline environment. A purple-colored reaction product is formed by the chelation of two molecules of BCA with one cuprous ion (Cu+1). Absorbance is detected at 562 nm using a fluorescence microplate reader. Protein concentration is determined using a BSA (bovine serum albumin) standard curve.

### F) Benzonase ELISA

The residual amount of benzonase was tested in an enzyme immunoassay, using the Benzonase ELISA II kit supplied by Merck (product number 1.01681). The kit uses specific polyclonal antibodies to capture benzonase, if present.

### G) Transmission electron microscopy to measure total virus particles

Transmission electron microscopy was performed to detect the total number of SFV viral particles (both infectious and non-infectious). In addition, this method enables assessing the size of the viral particles. The assay is performed by mixing virus particles with a standard quantity of latex beads. The mixture is then fixed on a grid and visualized using a transmission electron microscope. Latex beads and virus particles are counted and the concentration of virus particles is calculated based on the known concentration of latex beads.

### H) Heterogeneity (calculation)

The ratio of total virus particles versus infectious particles is calculated based on the results of the viral titer assay and the transmission electron microscopy. The ratio is an indication of the biological activity of the virus particles, i.e. when the ratio is 1 or lower, this indicates that all virus particles are infectious.

### Test results:

The results of the purification of 13 pooled harvest runs are shown in Table 1. It demonstrates that purification process as described in Example 2 efficiently removes Vero cell proteins and DNA and other process-related impurities such as Benzonase (As further described in Example 4 and Table 2). The ratio of 0.27 of the total: infectious particles demonstrated proficient biological activity of the purified SFV preparation. Given the variation of both assays, a ratio of 0.27 reflects that all physical particles are infectious. Importantly, the final titer after purification was sufficient for preparation of the final sterile formulation for dosing in humans.

**Table 1: Characterization of intermediate products and final preparation of SFV replicon particles.**

| Table 1A: Characterization of pooled harvest and QA load of 13 batches. | | | | | | | | | | | | | |
|---|---|---|---|---|---|---|---|---|---|---|---|---|---|
| **Test** | **16A26 -012** | **16B01 -005** | **16B02 -008** | **16B08 -025** | **16B08 -027** | **16B15 -012** | **16B15 -014** | **16B15 -016** | **16B29 -036** | **16B29 -034** | **16B29 -035** | **16B29 -037** | **16B29 -038** |
| **Crude pooled harvest** | | | | | | | | | | | | | |
| Viral titer (infectious particles/mL) | 2.0*10⁸ | 1.9*10⁸ | 1.9*10⁸ | 2.0*10⁸ | 1.6*10⁸ | 1.8*10⁸ | 1.0*10⁸ | 6.5*10⁷ | 1.7*10⁸ | 2.3*10⁸ | 9.3*10⁷ | 1.3*10⁸ | 1.1*108 |
| Purity: size residual DNA fragments | ND | ND | ND | ND | ND | ND | ND | ND | ND | ND | ND | ND | ND |

| **QA load** | | | | | | | | | | | | | |
|---|---|---|---|---|---|---|---|---|---|---|---|---|---|
| Viral titer (infectious particles/mL) | 6.9*10⁷ | 4.2*10⁷ | 4.2*10⁷ | 5.0*10⁷ | 3.5*10⁷ | 2.8*10⁷ | 1.9*10⁷ | 1.6*10⁷ | 3.9*10⁷ | 4.9*10⁷ | 1.7*10⁷ | 2.6*10⁷ | 2.7*10⁷ |

| | | | | | | | | | | | | | |
|---|---|---|---|---|---|---|---|---|---|---|---|---|---|
| ND: not detectable | | | | | | | | | | | | | |

**Table 1B: Characterization of the QA peak fraction, SO3 load, and SO3 peak fraction of 13 batches.**

| **Test** | **16A26 -012** | **16B01 -005** | **16B02 -008** | **16B08 -025** | **16B08 -027** | **16B15 -012** | **16B15 -014** | **16B15 -016** | **16B29 -036** | **16B29 -034** | **16B29 -035** | **16B29 -037** | **16B29 -038** |
|---|---|---|---|---|---|---|---|---|---|---|---|---|---|
| **QA peak fraction** | | | | | | | | | | | | | |
| Viral titer (infectious particles/mL)¹ | 1.5*10⁸ | 9.5*10⁷ | 1.1*108 | 1.6*10⁸ | 1.1*10⁸ | 1.6*108 | 8.2 *10⁷ | 5.5 *10⁷ | 1.7*10⁸ | 1.6*108 | 8.6*10⁷ | 8.1*10⁷ | 1.1*108 |
| Purity: total proteins (µg/mL) | 172 | 213 | 188 | 220 | 211 | 206 | 242 | 254 | 211 | 210 | 196 | 171 | 216 |
| Purity: host cell DNA (ng/mL) | 5 | 6 | 6 | 7 | 8 | 7 | 8 | 7 | 14 | 13 | 9 | 9 | 11 |

| **SO3 load** | | | | | | | | | | | | | |
|---|---|---|---|---|---|---|---|---|---|---|---|---|---|
| Viral titer (infectious particles/mL) | 2.1*10⁷ | 1.9*10⁷ | 1.7*10⁷ | 2.6*10⁷ | 2.0*10⁷ | 2.3*10⁷ | 1.4*10⁷ | 9.6*10⁶ | 2.7*10⁷ | 2.9*10⁷ | 1.4*10⁷ | 1.2*10⁷ | 1.8*10⁷ |

| **SO3 peak fraction** | | | | | | | | | | | | | |
|---|---|---|---|---|---|---|---|---|---|---|---|---|---|
| Purity: total proteins(µg/mL) | 19 | 20 | 16 | 27 | 23 | 8 | 27 | 29 | 26 | 22 | 15 | 19 | 28 |
| Purity: Vero host cell protein (µg/mL) | 0.84 | | | | | | | | | | | | |
| Purity: host cell DNA (ng/mL) | 2 | 3 | 4 | 4 | 5 | 3 | 3 | 3 | 7 | 5 | 4 | 4 | 6 |
| Total virus particles (total virus particles/mL) | 1.1*10⁸ | | | | | | | | | | | | |
| Viral titer (infectious particles/mL) after HSA addition | 4.2* 10⁸ | 6.4* 10⁸ | 4.4*10⁸ | 5.9*10⁸ | 4.1*10⁸ | 7.6*10⁷ | 4.3*10⁸ | 2.3*10⁸ | 5.8*10⁸ | 4.1*10⁸ | 2.8*10⁸ | 2.7*10⁸ | 4.8*10⁸ |
| Purity: total particles/ infectious particles ratio | 0.27 | | | | | | | | | | | | |

**Table 2: Intermediate yields and overall yield of infectious viral particles (in percentage compared to crude pooled harvest set at 100) of representative exemplary batches subjected to the two-step chromatographic purification method.**

| **Batch Number** | **Crude pooled harvest** | **QA load** | **QA peak fraction** | **SO3 load** | **SO3 peak fraction** |
|---|---|---|---|---|---|
| **16A26-012** | 100 | 136 | 112 | 64 | 22 |
| **16B01-005** | 100 | 79 | 49 | 40 | 22 |
| **16B15-014** | 100 | 69 | 57 | 39 | 22 |
| **16B29-038** | 100 | 97 | 66 | 42 | 20 |

### EXAMPLE 4 : Preparation of sterile composition comprising SFV particles

The fractions that were collected from the SO3 cation exchange column according to Example 2 and depicted in Table 1 were pooled, and the pool was split in two portions and each portion was separately processed. They were diluted 2.5- fold with water for injection (WFI) containing 1 w/v % HSA, to a target concentration of 1.56x10⁸ infectious particles/mL with formulation buffer. This resulted in two formulated drug substances 16E04-021 and 16H25-017, with a target concentration of 1.56x10⁸ infectious particles/mL in 240 mM NaCl, 20 mM HEPES pH 7.0,1 w/v % HSA. Both formulations were sterile filtered using a SLGP033RS Millex-GP Syringe Filter Unit, 0.22 µm, polyether sulfone, 33 mm, resulting in two clinical final product batches referred to as 16E8-011 and 16H25-004, respectively. In addition to the assays described in Example 3, the following methods were used to analyze the formulated drug substance and drug products:

### Biological activity and identity by Western Blot E6E7

The identity of the recombinant E6E7 SFV virus in the formulated product was determined using Western Blot analysis using standard procedures. Briefly, BHK21 cells were infected with the recombinant SFV virus at different MOI (multiplicity of infection). BHK21cells were lysed after 24 hours of incubation and the cell lysate was analyzed for E7 expression by Western Blot gel electrophoresis, using a mouse-anti-HPV16-E7 primary antibody and a goat-antimouse-AP (alkaline phosphatase) secondary antibody. Finally, E7 containing bands on the Western Blot are visualized by addition of an AP substrate, resulting in a chemiluminescent reaction.

### Absence of replication competent viruses

Replication competent viruses were detected using a cell based assay. Vero cells were seeded in roller bottles to allow detection of a single replication competent virus in the maximum human dose. Spiking studies demonstrated that replication competent viruses outgrow the replication incompetent virus rapidly causing cytopathic effects and destruction of the Vero cells. After sufficient Vero cells have been obtained the product is inoculated into the roller bottles and incubated for 14 days. Within the culture period, the cells are passaged once. Cells were evaluated for cytopathic effects at the end of the incubation period, which is indicative for the presence of replication competent viruses.

### Bioburden, pH, Osmolality, Appearance, Endotoxin, Visible particles and Extractable volume.

These are compendial methods that are described in the European Pharmacopeia (Ph. Eur.)

### Test results:

The characterization of these batches show that infectious titers before (Table 3) and after (Table 4) sterile filtration are similar (within the variation of the assay) demonstrating that no loss of virus had occurred during the filtration step. Furthermore, both batches met the release criteria for use in clinical trials.

**Table 3: Characteristics of exemplary formulated drug substance SFV replicon particle batches 16E04-021 and 16H25-004.**

| **Test** | **Specification** | **16E04-021** | **16H25-004** |
|---|---|---|---|
| Virus identity | Confirmed ID | Confirmed ID | Confirmed ID |
| Viral titer | 1.25x10⁸ ± 0.3log | 1.9x10⁸ | 1.9x10⁸ |
| Human serum albumin | 10 mg/mL ± 20% | 11 mg/mL | 11 mg/mL |
| Bioburden | ≤ 1 CFU/mL | 0 CFU/mL | 0 CFU/mL |
| Biological activity/ identity E6E7 | Protein expressed | Expressed | Expressed |
| pH | 7.0 ± 0.3 | 7.0 | 6.9 |
| Osmolality | 390 ± 40 mOsmol/kg | 395 mOsmol/kg | 347 mOsmol/kg |
| Appearance | Clear or slightly opalescent solution | Conform | conform |
| Absence of replication competent viruses | Not present in one human dose | Conform | Conform *⁵ |
| Benzonase | Not detected LOD=0.2 ng/mL | not detected | not detected * |

| | | | |
|---|---|---|---|
| *Tests on absence of replication competent viruses and on benzonase were performed on representative samples of formulated drug substance (lot # 16E04-021) | | | |

**Table 4: Characterization of exemplary formulated drug product SFV replicon particle batches 16E8-011 and 16H25-004 in HEPES, generated by sterile filtration of drug substance batches 16E04-021 and 16H25-004 and filling into glass vials.**

| **Test** | **Specification** | **16E18-011** | **16H25-004** |
|---|---|---|---|
| Sterility (Ph. Eur.) | no growth | no growth | no growth |
| Endotoxin concentration (Ph. Eur.) | <5 EU/mL | <5 EU/mL | <5 EU/mL |
| Visible particles (Ph. Eur.) | Essentially free of visible particles | Conforms | Conforms |
| Extractable volume (Ph. Eur.) | ≥ 1 mL/vial | Conforms | Conforms |
| Viral titer (infectious particles/mL) | 1.25x10⁸ ± 0.3log | 1.7x10⁸ | 1.6x10⁸ |
| Virus identity | Confirmed ID | Confirmed ID | Confirmed ID |
| Appearance (Ph. Eur visual inspection) | Clear or slightly opalescent solution | Conforms | Conforms |

### EXAMPLE 5: Comparison HEPES versus Tris buffer

To stabilize the virus particles during downstream processing, buffering components are used to maintain a relatively constant pH. In this example, the suitability of two buffer systems, i.e. HEPES and Tris, in downstream purification of SFV viral particles according to the invention was compared. To this end, small scale purification runs were performed; 2 parallel runs on CIM QA discs using HEPES buffer, and 2 parallel runs using Tris buffer were performed. In addition, 2 parallel runs on CIM SO3 discs using HEPES buffer, and 2 parallel runs using Tris buffer were performed.

First, SFV crude harvest was split in two halves, one halve was diluted with 50 mM HEPES pH 7.0 and the other halve with 50 mM Tris pH 7.0. Between 3.5-4.2x10⁸ infectious particles of the diluted crude harvest were loaded on each disc. The CIM QA discs were washed with either 50 mM HEPES pH 7.0, or 50 mM Tris pH 7.0. Thereafter, virus was eluted using either 50 mM HEPES pH 7.0 containing 0.3 M NaCl, or 50 mM Tris pH 7.0 containing 0.3 M NaCl. The CIM SO3 discs were washed with either 50 mM HEPES pH 7.0 containing 0.3 M NaCl, or 50 mM Tris pH 7.0 containing 0.3 M NaCl. Thereafter, virus was eluted using either 50 mM HEPES pH 7.0 containing 0.6 M NaCl, or 50 mM Tris pH 7.0 containing 0.6 M NaCl.

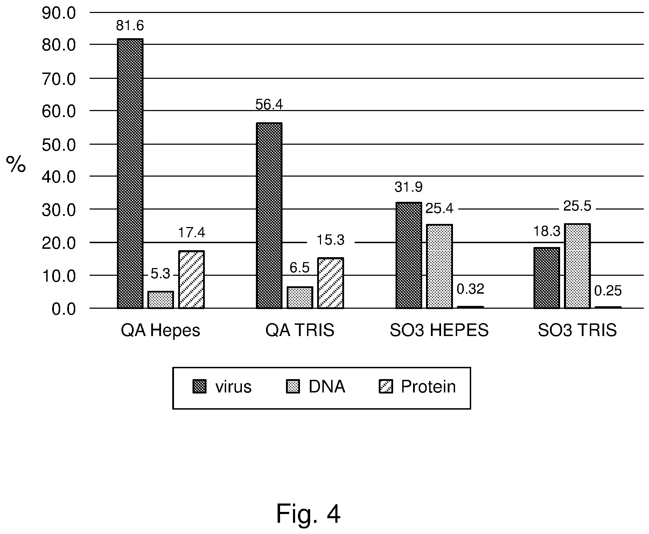

The average % virus yield of the two parallel runs, as well as the % of total protein and host cell DNA are shown in Figure 4. These results surprisingly show that, whereas the removal of protein and DNA impurities is similar using HEPES and Tris buffers, the SFV particle yields using the Zwitterionic HEPES buffer on the first (QA; 81.6%) and second (SO3; (31.9%) column is substantially higher than those using the non-zwitterionic Tris buffer ( 51.1 and 28.1%, respectively).

### EXAMPLE 6: Impact of conductivity on recovery of virus from the anion exchange column.

To evaluate the impact of the conductivity of the crude SFV replicon particle preparation on the recovery from the anion exchange column, a small scale experiment was performed using CIM^{®} QA 0.34 ml disks. To this end 3 parallel runs were performed:
- RUN 1 - 40 ml of SFV crude harvest was diluted with 50 mM HEPES pH 7.0 to final conductivity of 4 mS/cm and loaded onto CIM^{®} QA 0.34 ml disk
- RUN 2 - as in RUN 1 but diluted to final conductivity of 5 mS/cm
- RUN 3 - as in RUN 1 but diluted to a final conductivity of 9 mS/cm.

After loading, the column was washed with 50 mM HEPES pH 7.0, and SFV particles were eluted with 50 mM Tris pH 7.0 containing 0.3 M NaCl and collected in two fractions, E1 and E2, where E1 contains the main viral peak. The titer of the loaded virus, flow through/wash, E1 and E2 fractions were titrated for infectious viral particles (Table 5). The results demonstrate that the conductivity of 5 mS/cm provides the highest recovery in the E1 fraction. The recovery using a conductivity of 4 mS/cm was very similar, but the recovery dropped while using a conductivity of 9 mS/cm. Hence, for optimal recovery in the QA column the conductivity is preferably in a range between 4-5.5 mS/cm, more specifically 4.5 - 5.0 mS/cm.

**Table 5: Results of SFV particle purification CIM QA column using buffers with different conductivity (Runs 1-3). The viral titer in terms of infectious particles and recovery of total infectious virus particles in the different samples during purification are shown. FT= Flow through. E1 and E2 are elution fractions.**

| **Run 1** | ***Sample*** | ***Volume (ml)*** | ***Titer*/*ml*** | ***Total amount of virus particles*** | ***Recovery (%)*** |
|---|---|---|---|---|---|
| **4 mS/cm** | Original | 38,8 | 6,6E+07 | 2,6E+09 | |
| | Load | 177,7 | 1,5E+07 | 2,6E+09 | 103 |
| | FT | 177,7 | 2,0E+03 | 3,6E+05 | 0 |
| | Wash | 11,1 | 0,0E+00 | 0,0E+00 | 0 |
| | E1 | 8,6 | 2,0E+08 | 1,7E+09 | 68 |
| | E2 | 4,0 | 8,7E+07 | 3,5E+08 | 14 |
| | | | | | |
| | | | | | |

| **Run 2** | ***Sample*** | ***Volume (ml)*** | ***Titer*/*ml*** | ***Total amount of virus particles*** | ***Recovery (%)*** |
|---|---|---|---|---|---|
| **5 mS/cm** | Original | 41,0 | 6,6E+07 | 2,7E+09 | |
| | Load | 135,0 | 2,2E+07 | 2,9E+09 | 107 |
| | FT | 135,0 | 7,0E+04 | 9,5E+06 | 0 |
| | Wash | 10,4 | 0,0E+00 | 0,0E+00 | 0 |
| | E1 | 8,7 | 2,4E+08 | 2,1E+09 | 77 |
| | E2 | 4,0 | 8,5E+07 | 3,4E+08 | 13 |
| | | | | | |
| | | | | | |

| **Run 3** | ***Sample*** | ***Volume (ml)*** | ***Titer*/*ml*** | ***Total amount of virus particles*** | ***Recovery (%)*** |
|---|---|---|---|---|---|
| **9 mS/cm** | Original | 41,0 | 6,6E+07 | 2,7E+09 | |
| | Load | 70,0 | 3,6E+07 | 2,5E+09 | 92 |
| | FT | 70,0 | 1,7E+07 | 1,2E+09 | 43 |
| | Wash | 10,6 | 5,8E+05 | 6,1E+06 | 0 |
| | E1 | 8,8 | 1,4E+08 | 1,3E+09 | 47 |
| | E2 | 3,9 | 5,4E+07 | 2,1E+08 | 8 |

### EXAMPLE 6: Stability of SFV viral particles.

The stability of purified SFV replicon particles formulated into a clinical product as described in Examples 1-4, was tested up to 36 months storage at ≤ -60 °C. The product was tested at different time points for appearance, pH, Osmolality, and concentration of infectious virus particles. The data, as presented in Table 6, show that the clinical product is stable for at least 36 months in a 20 mM HEPES pH 7.0 buffer containing 1 w/v % HSA.

**Table 6: Stability results for SFV clinical drug product (batch 16E18-011) during storage at ≤ 60 °C.**

| **Test (and metho** | **Release specification** | **End of shelf life specification** | Storage period (months) | | | | | | |
|---|---|---|---|---|---|---|---|---|---|
| | | | **0** | **3** | **6** | **12** | **18** | **24** | **36** |
| Appearance (visual inspection) | Clear or slightly opalescent solution | Clear or slightly opalescent solution | Conforms | Conforms | Conforms | Conforms | Conforms | Conforms | Conforms |
| pH (Ph. Eur. 2.2.20) | 7.0 ± 0.3 | 7.0 ± 0.3 | 7.0 | n.a. | 7.0 | 7.0 | 7.0 | 7.0 | 7.0 |
| Osmolality (Ph. Eur. 2.2.35) | 390 ± 40 | 390 ± 40 | 342 | n.a. | 335 | 341 | 342 | 342 | 344 |
| In mOsmol/kg | | | | | | | | | |
| Viral titer (infectious particles/mL) (viral titer assay) | 1.25x10⁸ ± 0.3log10 | 1.25x10⁸ ± 0.3log10 | 1.6x10⁸ | 1.7x10⁸ | 1.8x10⁸ | 2.0x10⁸ | 1.8x10⁸ | 1.7x10⁸ | 1.4x10⁸ |
| Biological activity/ identity E6E7 (Western Blot) | Expression of protein confirmed | Expression protein confirmed | Conforms | n.a. | Conforms | Conforms | Conforms | Conforms | Conforms |
| | | no extra bands | | | | | | | |

## Claims

1. A method for providing purified viral replicon particles of Semliki Forest Virus (SFV), comprising the steps of
i) providing a preparation comprising crude SFV replicon particles;
ii) subjecting said preparation to an endonuclease treatment under conditions allowing for degradation of exogenous/host cell DNA and RNA;
iii) bringing said endonuclease-treated preparation with a zwitterionic buffer solution to a conductivity in the range of 4.5 - 5.0 mS/cm;
iv) contacting the preparation obtained in step (iii) with a strong anion exchange resin under conditions and for a time sufficient to bind to said resin, followed by washing the resin with a zwitterionic buffer solution to remove the portion of the preparation which does not bind to said anion exchange resin from said anion exchange resin;
v) eluting the bound SFV replicon particles from said anion exchange resin;
vi) bringing the eluted SFV replicon particles to a conductivity in the range of 7.5-8.2 mS/cm;
vii) contacting the preparation obtained in step (vi) with a strong cation exchange resin under conditions and for a time sufficient to bind to said resin, followed by washing the resin to remove the portion of the preparation which does not bind to said cation exchange resin from said cation exchange resin;
viii) eluting the bound SFV replicon particles from said cation exchange resin with a zwitterionic buffer solution and collecting at least one fraction containing purified SFV replicon particles
ix) stabilizing the at least one purified fraction by adding human serum albumin (HSA) to a final concentration in the range of 0.5 - 2 w/v%, preferably 1 w/v%.

2. Method according to claim 1, wherein step i) comprises providing a host cell that is modified to produce viral particles, culturing the modified host cell in a medium under conditions allowing expression of the structural proteins and replication of the SFV replicon nucleic acid, and then packaging of the SFV replicon nucleic acid to form SFV replicon particles.

3. Method according to claim 1 or 2, wherein step ii) comprises a benzonase treatment.

4. Method according to any one of the preceding claims, wherein the zwitterionic buffer solution used in at least one, preferably all, of the steps is selected from the group consisting of HEPES (N-2-hydroxyethyl piperazine-N'-2-ethanesulfonic acid), MOPSO (3-[N-morpholino]-2-hydroxy propanesulfonic acid), BES (N,N-bis (2-hydroxyethyl)-2-aminoethane sulfonic acid), and TAPSO (3-[N-tris(hydroxymethyl)methylamino]-2-hydroxy propanesulfonic acid).

5. Method according to claim 4, wherein the buffer is a HEPES buffer pH 7.0 ± 0.5, preferably a 40-60 mM HEPES buffer 7.0 ± 0.3.

6. Method according to any one of the preceding claims, wherein step iv) and/or step vii) comprise(s) the use of a monolithic chromatography column, preferably having an average pore diameter in the range of about 600-750 nm.

7. Method according to any one of the preceding claims, wherein the strong anion exchange resin is of the quaternary amine (QA) type.

8. Method according to any one of the preceding claims, wherein the strong cation exchange resin is of the sulfonyl/SO3 type.

9. Method according to any one of the preceding claims, further comprising performing a filtration or tangential flow filtration step in between steps iii) and iv).

10. Method according to any one of the preceding claims, wherein said SFV particles comprise a vector system comprising a nucleic acid molecule encoding a pathogen-derived antigen or a cancer-derived antigen.

11. Method according to claim 10, wherein the antigen is derived from a virus, bacterium, fungus or parasite, preferably wherein the virus is human papilloma virus (HPV), more preferably wherein the nucleic acid molecule encodes at least one antigenic polypeptide fragment of (HPV).

12. Method according to claim 11, wherein said polypeptide fragment is of a protein E6 or a protein E7 origin, preferably wherein said polypeptide fragment comprises an antigenic polypeptide fragment of the protein E6 and an antigenic polypeptide fragment of the protein E7.

13. A pharmaceutical composition comprising a stabilized preparation of purified SFV replicon particles and a pharmaceutically acceptable carrier, vehicle or diluent, wherein the particles show less than 0.3log10 change in viral titer upon ≥ 18 months storage at - 60 °C or lower, and wherein the composition is formulated with a HEPES buffer system at pH 7.0 ± 0.3, 200 - 250 mM NaCl, and 1-2 w/v% HSA in water for injection (WFI).

14. A pharmaceutical composition according to claim 13 for use in a method for vaccinating a subject, comprising administering to the subject an effective amount of said composition.

15. Pharmaceutical composition for use in a method according to claim 14, wherein said composition is administered by injection in at least a left and/or right limb, preferably in at least the right and left upper leg, of said subject.

## Patentansprüche

1. Verfahren zum Bereitstellen gereinigter viraler Replikonpartikel des Semliki Forest Virus (SFV), umfassend die folgenden Schritte
i) Bereitstellen eines Präparats, umfassend rohe SFV-Replikonpartikel;
ii) Unterziehen des Präparats einer Endonukleasebehandlung unter Bedingungen, die den Abbau von exogener/Wirtszell-DNA und -RNA ermöglichen;
iii) Bringen des mit Endonuklease behandelten Präparats mit einer zwitterionischen Pufferlösung auf eine Leitfähigkeit im Bereich von 4,5-5,0 mS/cm;
iv) Inkontaktbringen des in Schritt (iii) erhaltenen Präparats mit einem starken Anionenaustauscherharz unter Bedingungen und für eine Zeit, die ausreichen, um an das Harz zu binden, gefolgt von Waschen des Harzes mit einer zwitterionischen Pufferlösung, um den Abschnitt des Präparats, der nicht an das Anionenaustauscherharz bindet, von dem Anionenaustauscherharz zu entfernen;
v) Eluieren der gebundenen SFV-Replikonpartikel von dem Anionenaustauscherharz;
vi) Bringen der eluierten SFV-Replikonpartikel auf eine Leitfähigkeit im Bereich von 7,5-8,2 mS/cm;
vii) Inkontaktbringen des in Schritt (vi) erhaltenen Präparats mit einem starken Kationenaustauscherharz unter Bedingungen und für eine Zeit, die ausreichen, um an das Harz zu binden, gefolgt von Waschen des Harzes, um den Abschnitt des Präparats, der nicht an das Kationenaustauscherharz bindet, von dem Kationenaustauscherharz zu entfernen;
viii) Eluieren der gebundenen SFV-Replikonpartikel von dem Kationenaustauscherharz mit einer zwitterionischen Pufferlösung und Sammeln von mindestens einer Fraktion, die gereinigte SFV-Replikonpartikel enthält;
ix) Stabilisieren der mindestens einen gereinigten Fraktion durch Zugabe von Humanserumalbumin (HSA) auf eine Endkonzentration im Bereich von 0,5-2 Gew./Vol.-%, vorzugsweise 1 Gew./Vol-%.

2. Verfahren nach Anspruch 1, wobei Schritt i) das Bereitstellen einer Wirtszelle, die zur Bildung viraler Partikel modifiziert ist, das Kultivieren der modifizierten Wirtszelle in einem Medium unter Bedingungen, die die Expression der Strukturproteine und die Replikation der SFV-Replikonnukleinsäure erlauben, und dann das Verpacken der SFV-Replikonnukleinsäure zur Bildung von SFV- Replikonpartikeln umfasst.

3. Verfahren nach Anspruch 1 oder 2, wobei Schritt ii) eine Benzonasebehandlung umfasst.

4. Verfahren nach einem der vorstehenden Ansprüche, wobei die in mindestens einem, vorzugsweise allen, Schritten verwendete zwitterionische Pufferlösung ausgewählt ist aus der Gruppe, bestehend aus HEPES (N-2-Hydroxyethylpiperazin-N'-2-ethansulfonsäure), MOPSO (3-[N-Morpholino]-2-hydroxypropansulfonsäure), BES (N,N-Bis(2-hydroxyethyl)-2-aminoethansulfonsäure) und TAPSO (3-[N-tris(hydroxymethyl)methylamino]-2-hydroxypropansulfonsäure).

5. Verfahren nach Anspruch 4, wobei der Puffer ein HEPES-Puffer mit einem pH-Wert von 7,0 ± 0,5, vorzugsweise ein 40-60 mM HEPES-Puffer mit 7,0 ± 0,3 ist.

6. Verfahren nach einem der vorstehenden Ansprüche, wobei Schritt iv) und/oder Schritt vii) die Verwendung einer monolithischen Chromatographiesäule umfasst, die vorzugsweise einen durchschnittlichen Porendurchmesser im Bereich von etwa 600-750 nm aufweist.

7. Verfahren nach einem der vorstehenden Ansprüche, wobei das starke Anionenaustauscherharz vom Typ des quaternären Amins (QA) ist.

8. Verfahren nach einem der vorstehenden Ansprüche, wobei das starke Kationenaustauschharz vom Sulfonyl/SO3-Typ ist.

9. Verfahren nach einem der vorstehenden Ansprüche, ferner umfassend die Durchführung eines Filtrations- oder Tangentialflussfiltrationsschritts zwischen den Schritten iii) und iv).

10. Verfahren nach einem der vorstehenden Ansprüche, wobei die SFV-Partikel ein Vektorsystem umfassen, das ein Nukleinsäuremolekül umfasst, das ein von einem Pathogen abgeleitetes Antigen oder ein von Krebs abgeleitetes Antigen codiert.

11. Verfahren nach Anspruch 10, wobei das Antigen von einem Virus, einem Bakterium, einem Pilz oder einem Parasiten abgeleitet ist, wobei es sich bei dem Virus vorzugsweise um das humane Papillomavirus (HPV) handelt, wobei das Nukleinsäuremolekül mindestens ein antigenes Polypeptidfragment von (HPV) codiert.

12. Verfahren nach Anspruch 11, wobei das Polypeptidfragment von einem Protein E6 oder einem Protein E7 stammt, vorzugsweise wobei das Polypeptidfragment ein antigenes Polypeptidfragment des Proteins E6 und ein antigenes Polypeptidfragment des Proteins E7 umfasst.

13. Pharmazeutische Zusammensetzung, umfassend ein stabilisiertes Präparat von gereinigten SFV-Replikonpartikeln und einen pharmazeutisch unbedenklichen Träger, Vehikel oder Verdünnungsmittel, wobei die Partikel weniger als 0,31og10 Veränderung im viralen Titer nach ≥ 18 Monaten Lagerung bei -60 °C oder niedriger zeigen, und wobei die Zusammensetzung mit einem HEPES-Puffersystem bei pH 7,0 ± 0,3, 200-250 mM NaCl und 1-2 Gew./Vol.-% HSA in Wasser zur Injektion (WFI) formuliert ist.

14. Pharmazeutische Zusammensetzung nach Anspruch 13 zur Verwendung in einem Verfahren zur Impfung eines Subjekts, umfassend die Verabreichung einer wirksamen Menge der Zusammensetzung an das Subjekt.

15. Pharmazeutische Zusammensetzung zur Verwendung in einem Verfahren nach Anspruch 14, wobei die Zusammensetzung durch Injektion in mindestens eine linke und/oder rechte Extremität, vorzugsweise in mindestens den rechten und linken Oberschenkel, des Subjekts verabreicht wird.

## Revendications

1. Un procédé pour fournir des particules de réplicon viral purifié du virus de la forêt de Semliki (SFV), comprenant les étapes consistant à
i) fournir une préparation comprenant des particules de réplicon de SFV brutes ;
ii) soumettre ladite préparation à un traitement par endonucléase dans des conditions permettant la dégradation de l'ADN et de l'ARN de la cellule exogène/hôte ;
iii) amener ladite préparation traitée par endonucléase avec une solution tampon zwitterionique présentant une conductivité dans la plage de 4,5 à 5,0 mS/cm ;
iv) mettre en contact la préparation obtenue à l'étape (iii) avec une résine échangeuse d'anions forte dans des conditions et pendant une durée suffisante pour que la préparation se lie à ladite résine, suivie d'un lavage de la résine avec une solution tampon zwitterionique pour éliminer la partie de la préparation qui ne se lie pas à ladite résine échangeuse d'anions de ladite résine échangeuse d'anions ;
v) éluer les particules de réplicon SFV liées de ladite résine échangeuse d'anions
vi) amener les particules de réplicon SFV éluées présentant une conductivité comprise entre 7,5 et 8,2 mS/cm ;
vii) mettre en contact la préparation obtenue à l'étape (vi) avec une résine échangeuse de cations forte dans des conditions et pendant une durée suffisante pour se lier à ladite résine, suivi du lavage de la résine pour éliminer la partie de la préparation qui ne se lie pas à ladite résine échangeuse de cations de ladite résine échangeuse de cations ;
viii) l'élution des particules de réplicon de SFV liées de ladite résine échangeuse de cations avec une solution tampon zwitterionique et la collecte d'au moins une fraction contenant des particules de réplicon de SFV purifiées ;
ix) la stabilisation de la au moins une fraction purifiée par ajout de l'albumine de sérum humain (HSA) à une concentration finale dans la plage de 0,5 à 2 % p/v, de préférence 1% p/v.

2. Procédé selon la revendication 1, dans lequel l'étape i) comprend la fourniture d'une cellule hôte qui est modifiée pour produire des particules virales, la culture de la cellule hôte modifiée dans un milieu dans des conditions permettant l'expression des protéines structurales et la réplication de l'acide nucléique du réplicon du SFV, puis l'encapsidation de l'acide nucléique du réplicon du SFV pour former des particules de réplicon du SFV.

3. Procédé selon la revendication 1 ou 2, dans lequel l'étape ii) comprend un traitement à la benzonase.

4. Procédé selon l'une quelconque des revendications précédentes, dans lequel la solution tampon zwitterionique utilisée dans au moins une, de préférence toutes, les étapes est choisie dans le groupe constitué par l'HEPES (acide N-2-hydroxyéthyl pipérazine-N'-2-éthanesulfonique), le MOPSO (acide 3-[N-morpholino]-2-hydroxy propanesulfonique), le BES (acide N,N-bis(2-hydroxyéthyl)-2-aminoéthane sulfonique) et le TAPSO (acide 3-[N-tris(hydroxyméthyl)méthylamino]-2-hydroxy propanesulfonique).

5. Procédé selon la revendication 4, dans lequel le tampon est un tampon HEPES de pH 7,0 ± 0,5, de préférence un tampon HEPES 40-60 mM de pH 7,0 ± 0,3.

6. Procédé selon l'une quelconque des revendications précédentes, dans lequel l'étape iv) et/ou l'étape vii) comprend/comprennent l'utilisation d'une colonne de chromatographie monolithique, présentant de préférence un diamètre de pore moyen compris entre environ 600 et 750 nm.

7. Procédé selon l'une quelconque des revendications précédentes, dans lequel la résine échangeuse d'anions forte est du type amine quaternaire (QA).

8. Procédé selon l'une quelconque des revendications précédentes, dans lequel la résine échangeuse de cations forte est du type sulfonyle/SO3.

9. Méthode selon l'une quelconque des revendications précédentes, comprenant en outre la réalisation d'une étape de filtration ou de filtration à flux tangentiel entre les étapes iii) et iv).

10. Procédé selon l'une quelconque des revendications précédentes, dans lequel lesdites particules de SFV comprennent un système de vecteur comprenant une molécule d'acide nucléique codant pour un antigène dérivé d'un agent pathogène ou un antigène dérivé d'un cancer.

11. Procédé selon la revendication 10, dans lequel l'antigène est dérivé d'un virus, d'une bactérie, d'un champignon ou d'un parasite, de préférence dans lequel le virus est le papillomavirus humain (HPV), plus préférablement dans lequel la molécule d'acide nucléique code pour au moins un fragment de polypeptide antigénique de (HPV).

12. Procédé selon la revendication 11, dans lequel ledit fragment polypeptidique a pour origine une protéine E6 ou protéine E7, de préférence dans lequel ledit fragment polypeptidique comprend un fragment polypeptidique antigénique de la protéine E6 et un fragment polypeptidique antigénique de la protéine E7.

13. Une composition pharmaceutique comprenant une préparation stabilisée de particules de réplicon de SFV purifiées et un support, véhicule ou diluant pharmaceutiquement acceptable, dans laquelle les particules présentent une variation du titre viral inférieure à 0,3 log10 après un stockage ≥ 18 mois à -60 °C ou inférieur, et dans laquelle la composition est formulée avec un système tampon HEPES à pH de 7,0 ± 0,3, 200 - 250 mM de NaCl, et 1-2 % p/v de HSA dans de l'eau pour injection (WFI).

14. Composition pharmaceutique selon la revendication 13 pour une utilisation dans un procédé de vaccination d'un patient, comprenant l'administration au sujet d'une quantité efficace de ladite composition.

15. Composition pharmaceutique destinée à être utilisée dans un procédé selon la revendication 14, dans laquelle ladite composition est administrée par injection dans au moins un membre gauche et/ou droit, de préférence dans au moins dans la partie supérieure de la jambe droite et gauche dudit patient.
